# EUROPÄISCHE PATENTSCHRIFT

(11) **EP 1 084 146 B1**
(45) Veröffentlichungstag und Bekanntmachung des Hinweises auf die Patenterteilung: **13.11.2002**
(21) Anmeldenummer: 99950347.7
(22) Anmeldetag: 12.05.1999
(51) Int. Cl.: C07K 19/00, A61K 38/16

(54) **HYBRIDPROTEIN ZUR HEMMUNG DER MASTZELLDEGRANULATION UND DESSEN VERWENDUNG**
HYBRID PROTEIN FOR INHIBITING THE DEGRANULATION OF MASTOCYTES AND THE USE THEREOF
PROTEINE HYBRIDE POUR L'INHIBITION DE LA DEGRANULATION DES MASTOCYTES ET SON UTILISATION

(30) Priorität: 13.05.1998 DE 19821285
(43) Veröffentlichungstag der Anmeldung: 21.03.2001
(73) Patentinhaber: BioteCon Gesellschaft für Biotechnologische Entwicklung und Consulting mbH, 10589 Berlin (DE)
(72) Erfinder: BIGALKE, Hans, D-30419 Hannover (DE); FREVERT, Jürgen, D-10625 Berlin (DE)
(74) Vertreter: Dehmel, Albrecht, Dr.
(86) Internationale Anmeldenummer: EP9903272
(87) Internationale Veröffentlichungsnummer: WO99058571

(56) Entgegenhaltungen:
- WO-A-97/22364
- LUSTGARTEN, JOSEPH ET AL: "Prolonged inhibition of IgE production in mice following treatment with a IgE-specific immunotoxin" MOL. IMMUNOL. (1996), 33(3), 245-251 , XP002124080
- SLATER, JAY E. ET AL: "IgE immunotoxins. Effect of an IgE -ricin A chain conjugate on rat skin histamine content" J. IMMUNOL. (1988), 140(3), 807-11 , XP002124081

## Beschreibung

### Hintergrund der Erfindung

Allergische Reaktionen vom Soforttyp sind dadurch gekennzeichnet, daß betroffene Patienten Antikörper vom IgE-Typ gegen Allergene (z.B. Pollen, Hausstaubmilben, Tierhaare) gebildet haben. Diese Antikörper zirkulieren nicht nur im Blut, sondern sind auch gebunden an gewebeständigen Zellen, die einen spezifischen Rezeptor für einen Teil des IgE-Moleküls, das Fc-Fragment, in der Plasmamembran besitzen (Fishman & Lorberboum-Galski, 1997; Hamawy, M.M. 1997). Zellen mit IgE-Rezeptoren sind ausschließlich Mastzellen und Basophile. Diese Zellen sind die Effektorzellen für die allergische Reaktion vom Soforttyp. Sie speichern Vesikel, welche vasoaktive Amine sowie Prostaglandine und Leukotrine (Arachidonsäurederivate) enthalten. Die Freisetzung dieser Substanzen, die zur Degranulation der Mastzellen führt, erfolgt über einen spezifischen und einen unspezifischen Mechanismus. Werden Zellen mechanisch zerstört, z.B. durch einen Kratzer auf der Haut, wird Histamin unspezifisch freigesetzt. An der Wunde rötet sich die Haut. Es bilden sich Quaddeln (Ödeme) und die Haut juckt (triple response). Substanzen, die Histamin spezifisch freisetzen, sind in relativ geringen Konzentrationen wirksam und setzen folgende Kette von Ereignissen (Signalkaskade) in Gang: Aktivierung von Phospholipase C, - Bildung der second messenger 'Diacylglycerol' und 'IP3' - Mobilisierung von Kalzium aus zellulären Speichern - Fusion der Granula mit der Zellmembren - Exozytose der Granula ohne Zytolyse - Austausch von Natrium gegen das positiv geladene Histamin aus dem Komplex mit Heparin und einem basischen Protein - Freisetzung des Histamins aus der Granulamatrix. Kommt es zum Kontakt zwischen den Mastzellen eines Allergikers und einem Allergen, so binden die IgE-Moleküle auf der Zelloberfläche dieses Allergen. Werden genügend viele Allergenmoleküle gebunden, kommt es zur Aggregation der Rezeptoren in der Plasmamembran. Die Aggregation ist der spezifische Reiz für die Auslösung oben beschriebener Signalkaskade im Zellinneren. Die freigesetzten Substanzen lösen die allergischen Symptome aus (Konjunktivitis, Rhinitis, Asthma, Larynxödem, Urtikaria, Blutdruckabfall bis zum ausgeprägten anaphylaktischen Schock). Peptide, die im Gift von Bienen enthalten sind, wie z.B. das mast cell degranulating peptide (MCD), bewirken ebenfalls eine Mastzelldegranulation. Auch einige Arzneimittel verursachen eine spezifische Histaminfreisetzung als unerwünschte Wirkung. Beim Menschen ist die Histaminfreisetzung für Muskelrelaxantien, Dextrane, Azetylsalizylsäure (Aspirin), Morhpin, Antibiotika, Röntgenkontrastmittel, Fremdseren etc. beschrieben.

Gelingt es, die Verschmelzung der Vesikel mit der Plasmamembran zu hemmen, so unterbleibt die Ausschüttung der Amine und Arachidonsäurederivate. Es werden folglich keine allergischen Reaktionen ausgelöst. Am Sekretionsprozess bzw. der Freisetzung sind eine Reihe von Proteinen (Fusionsproteine) beteiligt, die an Membranen sekretorischer Vesikel und/oder an der Plasmamembran gebunden sein können. Sie können auch im Zytosol vorkommen. Zu diesen Proteinen gehören SNAP 25, Synaptobrevin (VAMP) und Syntaxin bzw. deren Isoformen. Diese Proteine bilden einen Komplex (Fusionskomplex), der die sekretorischen Vesikel an der Innenseite der Plasmamembran fixiert. Die Fixation geht der Verschmelzung der Vesikel mit der Plasmamembran voraus, die durch einen IgE-vermittelten Ca++-Einstrom ausgelöst wird. Durch die Inaktivierung eines der Proteine, etwa durch proteolytische Spaltung, wird die Bildung des Komplexes verhindert.

Bei Nervenzellen sind die genannten Fusionsproteine bekanntlich die Zielmoleküle (Substrate) der leichten Ketten der Neurotoxine, die vom Bazillus Clostridium botulinum produziert werden (Ahnert-Hilger & Bigalke, 1995). Bekannt sind derzeit sieben verschiedene Typen von Botulinumtoxinen (A,B,C1,D, E, F und G). Das erwähnte Synaptobrevin ist außerdem Zielmolekül für TeNT (Link et al., 1993), das von Clostridium tetani produziert wird, sowie für eine Protease aus Neisseria gonorrhoeae (Binscheck et al., 1995). Die Toxine, außer dem letztgenannten, bestehen aus mindestens zwei funktionellen Domänen. Der C-terminale Teil des Proteins (schwere Kette) ist verantwortlich für seine Bindung an die Nervenzelle, während der N-Terminus (leichte Kette) sich durch die oben beschriebene hochspezifische proteolytische Aktivität auszeichnet. Die Toxine binden über ihre schwere Kette an Nervenzellen und gelangen über eine rezeptorvermittelte Endozytose und nachfolgende Translokation ins Zytosol, wo sie eines oder mehrere der genannten Fusionsproteine spalten, die für den Fusionskomplex konstitutiv sind. Nach der Spaltung des jeweiligen Proteins ist die Sekretion von Azetylcholin bzw. anderer Transmitter aus den Nervenzellen gehemmt (Binscheck und Wellhöner, 1997).

Die Hemmung der Transmitterausschüttung wird bereits therapeutisch genutzt zur Behandlung dystoner Bewegungsstörungen sowie zur Unterdrückung überschießender parasympathischer Aktivitäten (Benecke und Kessler, 1995). Für die Clostridien-Neurotoxine sind keine anderen biologischen Substrate außer den Fusionsproteinen bekannt. Die schweren Ketten haben eine hohe Affinität zu peripheren Nervenzellen, so daß die mit ihnen verbundenen leichten Ketten nur in diese Zellen gelangen und in ihnen wirksam werden - obwohl auch andere Zelltypen, in denen Sekretionsprozesse stattfinden, die oben erwähnten Substrate, jedoch keinen Mechanismus zur Aufnahme der Protease besitzen (Marxen et al., 1989), beispielsweise Mastzellen und Basophile.

### Beschreibung der Erfindung

Eine Ausführungsform der Erfindung betrifft ein Hybridprotein, umfassend oder bestehend aus
(i) einem Protein, das an Mastzellen und/oder Basophile bindet und/oder von ihnen aufgenommen wird,
(ii) einer Protease, die ein oder mehrere Proteine des Sekretionsapparates der Mastzellen und/oder Basophilen spaltet.

Eine weitere Ausführungsform der Erfindung betrifft ein Hybridprotein, umfassend oder bestehend aus
(i) einem Protein, das an Mastzellen und/oder Basophile bindet und/oder von ihnen aufgenommen wird, wobei das Protein (i) aus der folgenden Gruppe ausgewählt ist:
   IgE;
      IgE-Fragment, insbesondere IgE-Fc-Fragment; Antikörper gegen IgE-Rezeptor von Mastzellen und/oder Basophilen;
      Fragment des Antikörpers gegen IgE-Rezeptor von Mastzellen und/oder Basophilen, insbesondere Fab-Fragment;
      Antikörper gegen mastzellspezifischen Kaliumkanal; und inaktives, jedoch bindendes MCD-Peptid, und
(ii) einer Protease, die ein oder mehrere Proteine des Sekretionsappa rates der Mastzellen und/oder Basophilen spaltet.

Eine weitere Ausführungsform der Erfindung betrifft ein Hybridprotein, umfassend oder bestehend aus
(i) einem Protein, das an Mastzellen und/oder Basophile bindet und/oder von ihnen aufgenommen wird, und
(ii) einer Protease, die ein oder mehrere Proteine des Sekretionsapparates der Mastzellen und/oder Basophilen spaltet, wobei die Protease (ii) aus der folgenden Gruppe ausgewählt ist:
   leichte Kette eines Clostridium botulinum-Toxins, insbesondere Typ A, B, Cl, D, E, F oder G;
   katalytisch aktives Fragment der leichten Kette eines Clostridium botulinum-Toxins, insbesondere Typ A, B, Cl, D, E, F oder G;
   leichte Kette von Tetanustoxin (TeNT);
   katalytisch aktives Fragment der leichten Kette von Tetanustoxin;
   IgA-Protease von Neisseria gonorrhoeae; und
   katalytische Domäne der IgA-Protease von Neisseria gonorrhoeae.

Das erfindungsgemäße Hybridprotein kann dadurch gekennzeichnet sein, daß das Protein (i) aus der vorstehenden Protein-Gruppe und die Protease (ii) aus der vorstehenden Protease-Gruppe ausgewählt ist.

Das erfindungsgemäße Hybridprotein kann ferner dadurch gekennzeichnet sein, daß zusätzlich zur leichten Kette eines Clostridium botulinum-Toxins oder von Tetanustoxin auch der N-terminale Teil der schweren Kette des betreffenden Toxins (H_{N}-Fragment) oder ein Fragment davon ein Teil des Hybridproteins ist.

Schließlich betrifft eine Ausführungsform der Erfindung die Verwendung eines erfindungsgemäßen Hybridproteins für die Herstellung eines Medikamentes zur Hemmung von Mastzellgranulation.

Würde man Mastzellen abtöten, so bestünde die Gefahr, daß es zu einem allergischen Schock kommt, wenn die absterbenden Mastzellen die gespeicherten endogenen Amine freisetzen. Weiterhin stimuliert der Abfall der Mastzellzahl die Neusynthese dieser Zellen, die dann erneut für allergische Reaktionen zur Verfügung stehen. Das erfindungsgemäße Hybridprotein unterscheidet sich damit fundamental von dem bekannten IgE-Fc/Pseudomonas-Exotoxin-Konjugat, das durch seine ADP-Ribosylierungs-Aktivität die Proteinsynthese inhibiert und damit den Zelltod bewirkt (Fishman & Lorberboum-Galski, 1997). Demgegenüber dient das erfindungsgemäße Hybridprotein nicht zur Abtötung von Mastzellen. Die Zellen bleiben vielmehr nach der Einwirkung vital und haben nur die Fähigkeit eingebüßt, vasokonstriktive Amine freizusetzen.

Eine Stimulation der Neusynthese unterbleibt. Bei einem therapeutischen Einsatz werden mögliche toxische Nebenwirkungen vermieden, die bei einem Konjugat zu erwarten sind, das auf dem kompletten cytotoxischen Pseudomonas-Toxin oder einem vergleichbaren Cytotoxin basiert.

Gegenstand der Erfindung kann also ein Konjugat (Hybrid-Protein) sein, bestehend aus (i) einem Protein oder Peptid (Transportprotein/-peptid) mit einer hohen Affinität zu Mastzellen/Basophilen und (ii) einer spezifischen Protease, welches die Degranulation bzw. den Sekretionsmechanismus der Zellen blockiert. Das Konjugat dient zur Therapie/Prophylaxe von allergischen Reaktionen vom Soforttyp.
(i) Als hochaffine, mastzellbindende Komponente des Konjugates werden insbesondere Immunglobulin vom Typ E (IgE) bzw. dessen Fragmente (z.B. das Fc-Fragment) verwendet. Des weiteren werden auch Antikörper gegen spezifische Oberflächenmoleküle von Mastzellen/Basophilen eingesetzt, welche selektiv an deren Plasmamembran binden. Vor allem Antikörper gegen den IgE-Rezeptor erfüllen diesen Zweck. Weiterhin sollen inaktive, aber bindende Mutanten des mast cell-degranulating peptide als Transportpeptid im Hybrid-Protein verwendet werden. Diese Transportpeptide/-proteine dienen dazu, eine Protease in die Zellen zu schleusen. Diese Protease spaltet im Fusionskomplex der Mastzellen hochspezifisch Proteine, die den Degranulationsmechanismus der Zellen einleiten.
(ii) Als hochspezifische Protease dient eine Metalloproteinase, z.B. die leichte Kette von Botulinumtoxin Typ A, B, C1, D, E, F oder G (BoNT/X) sowie von Tetanustoxin (TeNT) oder die IgA-Protease aus Neisseria gonorrhoeae. Diese Proteasen spalten das synaptosomal-associated protein (MR 25.000) (SNAP 25), Synaptobrevin oder Syntaxin. Ist nur eines der genannten Proteine/Peptide gespalten, ist die Degranulierung der Mastzellen gehemmt. Dann wird keine Sekretion von Histamin, Prostaglandinen und Leukotrienen mehr stattfinden, und allergische Symptome können nicht mehr auftreten.

In der vorliegenden Erfindung können die ungiftigen leichten Ketten der Toxine mit Transportproteinen verknüpft werden, die ausschließlich an Mastzellen bzw. Basophilen binden und daher nur von diesen aufgenommen werden, wobei die leichten Ketten - sozusagen als Trittbrettfahrer - zugleich in solche Zellen gelangen. In Nervenzellen oder andere Zelltypen des Organismus können sie nicht eindringen, so daß die Wirkung auf Mastzellen und Basophile beschränkt bleibt. Wenn eines der Substrate proteolytisch zerstört ist, treten nach dem Kontakt dieser IgE beladener Zellen mit einem Allergen oder einem der oben erwähnten Arzneimittel keine allergischen Symptome auf.

Als mastzellspezifisch bindende Proteine werden
1) Immunglobuline vom Typ E, deren Fragmente vom Typ Fc,
2) Antikörper gegen den IgE Rezeptor,
3) das mast cell-degranulating peptide und
4) ein Antikörper gegen den mastzellspezifischen Kaliumkanal verwendet.

Zu den aufgeführten Proteinen kann auf folgenden Stand der Technik verwiesen werden:
- IgE Helman (1995)
- IgE-Fc-Fragment Helman (1995)
- Antikörper gegen IgE-Rezeptor von Mastzellen/Basophilen, Antikörper gegen mastzellspezifischen Kaliumkanal, Fab-Fragment des Antikörpers: es handelt sich dabei um Standardverfahren, die beschrieben sind in:
   Liddel & Weeks (1995)
- MCD Peptid
   Gmachel & Krell (1995)
- Inaktive aber bindende Mutante
   Das mutierte Peptid wird nach Standardverfahren hergestellt:
   Nichol D.S.T. (1995)
- Leichte Ketten der verschiedenen Botulinumtoxine Typ A - G:
   Binz et al. (1990)
- Leichte Kette von Tetanustoxin
   Eisel et al. (1989)
- IgA-Protease
   Bruscheck et al. (1995)

Die Verknüpfung beider Komponenten (Transportprotein und Protease) erfolgt auf verschiedenem Wege:

Zunächst wird die leichte Kette des Toxins chromatographisch gereinigt. Die leichte Kette ist völlig untoxisch, weil sie nach Trennung von der schweren Kette, dem neurotropen Transportprotein, nicht in Nervenzellen gelangen kann und ein extrazelluläres Substrat nicht vorkommt. Die leichte Kette wird sodann chemisch mit einem der vier mastzellbindenden Proteine zu einem Konjugat verknüpft, welches in das Zytosol von Mastzellen aufgenommen wird. Dort spaltet die leichte Kette ihr Substrat, worauf die Sekretion von Histamin und anderer Substanzen blockiert ist. Ein zweiter Syntheseweg des Konjugats besteht darin, das Gen für die leichte Kette mit dem Gen für eines der vier mastzellbindenden Proteine zu fusionieren, so daß in geeigneten Wirtszellen ein Hybridprotein exprimiert wird. Dieses biotechnologisch produzierte Hybridprotein sollte analog zum aus zwei Proteinkomponenten hergestellten Konjugat den Sekretionsprozess aus Mastzellen blockieren.

Die Herstellung von Hybridproteinen an sich ist ein bekanntes Verfahren insbesondere im Bereich der Tumortherapie (Vogel, C.-W. 1987; Magerstadt, M., 1991). In diesem therapeutischen Konzept wird ein Antikörper gegen Oberflächenproteine der Tumorzellen mit einem cytotoxischen Protein, z.B. Ricin, Diphtherietoxin, verknüpft, um Krebszellen abzutöten. Neu an dem hier vorgestellten erfindungsgemäßen Verfahren ist der Einsatz von spezifischen Proteasen bzw. proteolytischen Domänen in Hybridproteinen zur Inhibition der Mastzelldegranulation und damit zur antiallergischen Therapie. Mit diesen Hybridproteinen ließen sich nicht nur stark beeinträchtigende allergische Symptome (Heuschnupfen, Asthma und Neurodermitis) verhindern. Sie könnten auch prophylaktisch zur Vermeidung allergischer Reaktionen im Rahmen von Therapien mit lebensrettenden Arzneimitteln verabreicht werden. Darüber hinaus könnten sie allergische Symptome verhindern, die bei Desensibilierungen auftreten.

### Beispiel 1: Synthese eines Hybridproteins aus IgE und der leichten Kette von BoHT/ A

Das gereinigte Botulinumtoxin (5.0mg) vom Typ A wurde nach Äquilibrierung in 15 mM Natriumtetraborat und 30 mM Phosphat, pH=8.4, auf eine mit dem gleichen Puffer äquilibrierte QAE-Sephadex-Säule (1.0x3.0 cm) aufgetragen. Die Säule wurde anschließend mit 10 mL 120 mM Dithioerythrol, 2 M Harnstoff und 1 mM EDTA gewaschen und über Nacht inkubiert. Danach wurde die leichte Kette mit 10 mM Boratpuffer von der Säule eluiert und gegen 20 mM Phosphat, pH=7.0, dialysiert.

Immunoglobulin E (Ratte) wurde käuflich erworben. 10 mg des Immunglobulins wurden mit 50µg Papain in 1 mL Phosphatpuffer gespalten (4°C über Nacht). Das F_{c-}Fragment wurde über ein Gelfiltrationssäule Sephacryl S200) gereinigt. 3.0 mg des gereinigten F_{c}-Fragments wurden mit 3.0 mg gereinigter leichter Kette von Botulinumtoxin mit dem 10mM bifunktionellen Agens Dithiobissuccinimidylpropionat in 2 mL Na-Phosphat, pH=7.0, 16 Stunden inkubiert. Das so synthetisierte Hybridprotein wurde durch Gelfiltration (Sephacryl S200) gereinigt und in der SDS-Gelektrophorese auf Reinheit analysiert.

Die Hemmung der Degranulation der Mastzellen wird in zwei Versuchsansätzen überprüft. Im ersten Ansatz werden isolierte Mastzellen aus der Ratte mit dem Hybridmolekül inkubiert, und anschließend wird die Histaminfreisetzung stimuliert. Die Stimulation erfolgt mit spezifischen Histaminliberatoren, wie dem MCD-Peptid und dem Concanavalin A (letzteres ist eine experimentell genutzte Substanz), bzw. durch direkte Erhöhung der intrazellulären Kalziumkonzentration. Letzteres erreicht man durch eine Injektion von Kalzium in einzelne Mastzellen. Damit schließt man die oben beschriebene Signalkaskade kurz, denn die Erhöhung der Kalziumkonzentration ist der Schritt im Sekretionsprozeß, dem die Vesikelfusion folgt. Die Degranulation der Mastzelle, die die Histaminfreisetzung widerspiegelt, wird im Phasenkontrastmikroskop verfolgt. Im weiteren kann freigesetztes Histamin mit Hilfe einer Fluoreszenzmessung quantifiziert werden. Schließlich kann die Vergrößerung der Mastzelle, die durch Einlagerung von Vesikelmembranen in die Plasmamembran bei der De-granulation verursacht wird, elektrophysiologisch bestimmt werden. In den mit Hybridprotein behandelten Zellen wird im Gegensatz zu Kontrollzellen 1. keine morphologische Änderung auftreten, 2. keine Verstärkung der Fluoreszenz im Zellüberstand stattfinden und 3. keine Vergrößerung der Zelle erfolgen. Damit kann nachgewiesen werden, daß die Freisetzung von Histamin durch das Hybridprotein blockiert ist.

In dem zweiten Ansatz wird das Hybridprotein lebenden Ratten injiziert. Die Ratten werden nach mehren Tagen getötet und ihre Mastzellen mit konventionellen Methoden gewonnen. Die Degranulation, bzw. Freisetzung von Histamin wird wie oben beschrieben bestimmt. In diesem Ansatz wird geprüft, ob das Konjugat auch im lebenden Tier in das Kompartiment gelangen kann, in dem sich die Mastzellen befinden, und ob es sie dort inaktiviert.

### Beispiel 2: Herstellung eines rekombinanten Hybridproteins durch Verknüpfung des Gens für die leichte Kette von Clostridium botulinum Typ A mit dem Gen für Immunglobulin E bzw. eines seiner Fragmente (Fc-Fragment.)

Das Gen für die leichte Kette von Botulinumtoxin A wird mittels geeigneter Primer über die PCR (polymerase chain reaction) isoliert. Dazu wird eine Kultur von Clostridium botulinum Typ A angelegt, aus der die DNA präpariert wird. Aus der publizierten Sequenz des Toxingens (Binz et al.) wird ein Primerpaar abgeleitet und mittels PCR das Gen für die leichte Untereinheit amplifiziert. Anschließend wird dieses Gen in einen kommerziellen Expressionsvektor pQE nach Angaben des Herstellers kloniert.

Das Gen für das F_{c}-Fragment des humanen Immunoglobulins E (Helman L.), wurde mittels PCR aus einer kommerziellen cDNA-Bank isoliert und im Vectorkonstrukt mit dem Gen für die leichte Kette von Botulinumtoxin A fusioniert.

Mit diesem Konstrukt werden kompetente M15-Zellen (E.coli) transfomiert. Da in diesem Expressionssystem die insertierten Gene mit einem "his-tag" versehen sind, wird das rekombinate Protein über eine Ni-Affinitätssäule gereinigt. Der Hochreinigung schließt sich eine Gelfiltration über Sephacryl S300 an.

Die Testung der bioloigschen Aktivität erfolgte wiederum an isolierten Mastzellen in vitro.

### Beispiel 3: Herstellung eines rekombinanten Hybridproteins durch Verknüpfung des Gens für die leichte Untereinheit von Tetanustoxin mit einem mutierten Gen für das Mast Cell Degranulating Peptide (MCD)

Die "Sequenz für das "Mast Cell Degranulating Peptide", ein 22mer, ist bekannt (Gmachl, M, und Kreil, G.) Darauf basierend wird ein korrespondierenes Oligonukleotid synthetisiert.

Zur Isolierung der Sequenz der leichten Untereinheit von Tetanustoxin wurde Kultur von C. tetani angelegt und daraus DNA gewonnen. Aus der bekannten Nukleinsäuresequenz von Tetanustoxin wurde ein Primer für die PCR und damit das Gen für die leichte Untereinheit des Toxins gewonnen.

Wie in Beispiel 1 beschrieben, wurden beide Nukleinsäuresequenzen in einem Expressionsvektor pQU fusioniert und dann in E. coli exprimiert. Das Hybridprotein, das wiederum mit einem histag versehen war, wurde durch Affinitätschrotographie und anschließende Gelfiltration gereinigt. Gereinigtes Gen für das Mast Cell Degranulating Peptide wird chemisch synthetisiert mit einer Punktmutation in der aktiven Domäne des Petids. Das Gen wird verknüpft mit dem Gen für die leichte Kette von Tetanustoxin. Das Hybridprotein wird in E. coli exprimiert und gereinigt. Das so hergestellte Hybridproptein wird in vitro im Mastzeil-Degranulations-Assay geprüft.

### Beispiel 4: Herstellung eines rekombinanten Hybridproteins durch Verknüpfung des Gens für das F_{c}-Fragment von IgE mit dem Gen für die IgA-Protease

Das Gen für das F_{c}-Fragment von IgE wurde, wie im Beispiel 1 beschrieben, isoliert.

Das Gen für die IgA-Protease aus N. gonorrhoeae ist bekannt. Daraus wurden Primer abgeleitet, und aus einer Nukleinsäurepräparation von N. gonorrhoeae wurde mittels PCR das Gen für die spezifische Protease gewonnen.

Beide Nukleinsäuren wurden in einen kommerziellen Vektor nach den Angaben des Herstellers integriert und das Hybridprotein durch Affinitätschromatographie (s. Beispiel 2) gereinigt.

Die inhibitorische Aktivität wird wiederum in vitro an isolierten Mastzellen nachgewiesen (s.o.)

### Beispiel 5: Herstellung eines Hybridproteins, das aus dem Fab-Fragment eines Antikörper gegen den IgE-Rezeptor und der leichten Kette von Botulinumtoxin Typ B besteht

Ein monoklonaler Antikörper gegen den IgE-Rezeptor auf Mastzellen wurde käuflich erworben und chromatographisch nachgereinigt. 0.5 mg des Antikörpers wurden mit 0.4 g der gereinigten leichten Kette von Botulinumtoxin F konjugiert. Die leichte Untereinheit wurde nach der Präparation des Neurotoxins nach dem gleichen Verfahren wie in Beispiel 1 beschrieben durch Spaltung des Neurotoxins und anschließender Reinigung über eine lonenaustasucherchromatographie isoliert.

Die beiden Proteine (leichte Untereinheit von Toxin Typ F und monoklonaler Antikörper) wurden mit einem bifunktionellen Reagens mit einander verknüpft. Dazu wurden die isolierten Proteine mit 10 mM Maleimidobenzoyl-N-hydroxy-succinimidester inkubiert. Das Hybridprotein wurde anschließend durch Gelfiltration über Sephacryl S300 von nichtkonjugierten Proteinen gereinigt.

An isolierten Mastzellen wird wiederum gezeigt, daß das synthetisierte Hybridprotein die Histaminsekretion hemmt.

Bezug zu anderen Patenten:
Patent No. 4902495
IgE Fc directed delivery systems

Novel Agent Controlling Cell Activity
PCT Anmeldung WO 94/21300

Literatur:
1) Ahnert-Hilger G., Bigalke H. (1995 )
   Molecular aspects of tetanus and botulinum neurotoxin poisoning. Progress in Neurobiology **46**: 83-96
2) Benecke R., Kessler K.R. (1995)
   Botulinumtoxin A
   Akt. Neurol. **22**: 209-213
3) Binscheck T., Bartels F., Bergel H., Bigalke H., Yamasaki S., Hayashi T.,
   Niemann H., Polner J. (1995)
   IgA protease from Neisseria gonorrhoeae inhibits exocytosis in bovine chromaffin cells like tetanus toxin.
   J. biol. Chem. **270:** 1770-1774
4) Binscheck T., Wellhöner H.H. (1997)
   Tetanus and botulinum toxins - zinc proteases - synaptotagmin - exocytosis.
   In: Toxins and signal transduction
   (Eds.: Gutman Y., Lazarovici P.) 1: 457-487
5) Binz, T. , Kurazono, H., M., Frevert, J. , Wernars,K. & Niemann, H. (1990)
   The complete sequence of botulinum toxin A and comparison with other clostridial neurotoxins
   J.Biol.Chem. **265**: 9153-9158
6) Cardoso, F. & Jancovic, J. (1995)
   Clinical use of botulinum neurotoxins
   Current Topics Microbiol.Imunol. **195**: 123-141
7) Fishman, A., Lorberboum-Galski, H. (1997)
   Targeted elimination of cells expressing the high-affinity receptor for IgE (Fc epsilon RI) by a Pseudomonas exotoxin-based chimeric protein
   Eur. J. Immunol., 27:486-494
8) Gmachl, M. & Kreil, G. (1995)
   The precursors of the bee venom constituents apamin and MCE peptide are encoded by two genes in tandem which share the same 3'-exon
   J.Biol.Chem. **270** (21): 12704-12708
9) Helman, L. (1995)
   Characteriziation of four novel epsilon chain mRNA and a comparative analysis of genes for immunoglobulin E in rodents and man
   Eur. J. Immunol. **23** (1):, 159-167
10) Liddel & Weeks (1995)
   Antikörper-Techniken
   Spektrum Akademischer Verlag Heidelberg
11) Link E., Edelmann 1., Chou J., Binz T., Yamasaki S., Eisel U., Baumert M.,
   Südhof T.C., Niemann H.& Jahn R. (1992)
   Tetanus toxin action: Inhibition of neurotransmitter release linked to synaptobrevin proteolysis.
   Biochem. Biophys. Res. Comm. **189:** 18423-26
12) Magerstadt, M. (1991)
   Antibody conjugates and malignant diseases
   CRC Press
13) Hamawy, M.M. (Ed.) 1997)
   IgE Receptor (FceRI) Function
   In: Mast Cells and Basophils
   Springer Verlag
14) Marxen P., Fuhrmann U., Bigalke H. (1989)
   Gangliosides mediate inhibitory effects of tetanus and botulinum A neurotoxins on exocytosis in chromaffin
   cells.
   Toxicon **27**: 849-859
15) Nichol D.S.T. (1995)
   Gentechnische Methoden
   Spektrum Akademischer Verlag
16) Vogel, C.-W. (Ed.) (1987)
   Immunoconjugates: Antibody conjugates in radio-immagining anth therapy of cancer
   Oxford UP Inc.

## Patentansprüche

1. Hybridprotein, umfassend oder bestehend aus
(i) einem Protein, das an Mastzellen und/oder Basophile bindet und/oder von ihnen aufgenommen wird, und
(ii) einer Protease, die ein oder mehrere Proteine des Sekretionsapparates der Mastzellen und/oder Basophilen spaltet.

2. Hybridprotein, umfassend oder bestehend aus
(i) einem Protein, das an Mastzellen und/oder Basophile bindet und/oder von ihnen aufgenommen wird, wobei das Protein (i) aus der folgenden Gruppe ausgewählt ist:
IgE, IgE-Fragment, insbesondere IgE-Fc-Fragment,
Antikörper gegen IgE-Rezeptor von Mastzellen und/oder Basophilen, Fragment des Antikörpers gegen IgE-Rezeptor von Mastzellen und/oder Basophilen, insbesondere Fab-Fragment,
Antikörper gegen mastzellspezifischen Kaliumkan und inaktives, jedoch bindendes MCD-Peptid, und
(ii) einer Protease, die ein oder mehrere Proteine des Sekretionsapparates der Mastzellen und/oder Basophilen spaltet.

3. Hybridprotein, umfassend oder bestehend aus
(i) einem Protein, das an Mastzellen und/oder Basophile bindet und/oder von ihnen aufgenommen wird, und
(ii) einer Protease, die ein oder mehrere Proteine des Sekretionsapparates der Mastzellen und/oder Basophilen spaltet, wobei die Protease (ii) aus der folgenden Gruppe ausgewählt ist:
leichte Kette eines Clostridium botulinum-Toxins, insbesondere Typ A, B, C1, D, E, F oder G;
katalytisch aktives Fragment der leichten Kette eines Clostridium botulinum-Toxins, insbesondere Typ A, B, C1, D, E, F oder G;
leichte Kette von Tetanustoxin (TeNT);
katalytisch aktives Fragment der leichten Kette von Tetanustoxin (TeNT);
IgA-Protease von Neisseria gonorrhoeae; und
katalytische Domäne der IgA-Protease von Neisseria gonorrhoeae.

4. Hybridprotein nach Anspruch 2 oder 3, **dadurch gekennzeichnet, daß** das Protein (i) aus der Gruppe gemäß Anspruch 2 und die Protease (ii) aus der Gruppe gemäß Anspruch 3 ausgewählt ist.

5. Hybridprotein nach Anspruch 4, **dadurch gekennzeichnet, daß** zusätzlich zur leichten Kette eines Clostridium botulinum-Toxins oder von Tetanustoxin auch der N-terminale Teil der schweren Kette des betreffenden Toxins (H_{N}-Fragment) oder ein Fragment davon ein Teil des Hybridproteins ist.

6. Hybridprotein nach einem der vorhergehenden Ansprüche zur Verwendung als Arzneimittel in der Humanund Tiermedizin.

7. Verwendung des Hybridproteins nach einem der Ansprüche 1 bis 5 für die Herstellung eines Medikamentes zur Hemmung von Mastzelldegranulation.

## Claims

1. Hybrid protein comprising or consisting of
(i) a protein binding to mastocytes and/or basophils and/or being taken up by these cells,
(ii) a protease cleaving one or several proteins of the secretion apparatus of the mastocytes and/or basophils.

2. Hybrid protein comprising or consisting of
(i) a protein binding to mastocytes and/or basophils and/or being taken up by these cells, wherein the protein (i) is selected from the group consisting of:
IgE;
IgE fragment, in particular IgE Fc fragment;
antibody against IgE receptor of mastocytes and/or basophils; fragment of the antibody against IgE receptor of mastocytes and/or basophils, in particular Fab fragment; antibody against mastocyte-specific potassium channel; and
inactive but binding MCD peptide; and
(ii) a protease cleaving one or several proteins of the secretion apparatus of the mastocytes and/or basophils.

3. Hybrid protein comprising or consisting of
(i) a protein binding to mastocytes and/or basophils and/or being taken up by these cells; and
(ii) a protease cleaving one or several proteins of the secretion apparatus of the mastocytes and/or basophils, wherein the protease (ii) is selected from the group consisting of:
light chain of a *Clostridium botulinum* toxin, in particular the toxins of type A, B, C1, D, E, F, and G;
catalytically active fragment of the light chain of a *Clostridium botulinum* toxin, in
particular a toxin of type A, B, C1, D, E, F, and G;
light chain of the tetanus toxin (TeNT);
catalytically active fragment of the light chain of the tetanus toxin;
IgA protease of *Neisseria gonorrhoeae;* and
catalytic domain of the IgA protease of *Neisseria gonorrhoeae.*

4. Hybrid protein of claim 2 or 3, **characterized in that** the protein (i) is selected from the group according to claim 2 and the protease (ii) is selected from the group according to claim 3.

5. The hybrid protein of claim 4, **characterized in that** the N-terminal portion of the heavy chain of the respective toxin (H_{N} fragment) or a fragment thereof is a portion of the hybrid protein, in addition to the light chain of a *Clostridium botulinum* toxin or of the tetanus toxin.

6. Hybrid protein of any of the preceding claims for use as a pharmaceutical in human and veterinary medicine.

7. Use of the hybrid protein of any of claims 1 to 5 for the manufacture of a medicament for the inhibition of the degranulation of mastocytes.

## Revendications

1. Protéine hybride comprenant ou consistant en :
(i) une protéine se liant aux mastocytes et/ou aux basophiles et/ou étant absorbée par eux, et
(ii) une protéase clivant une ou plusieurs protéines de l'appareil de sécrétion des mastocytes et/ou des basophiles.

2. Protéine hybride comprenant ou consistant en :
(i) une protéine se liant aux mastocytes et/ou aux basophiles et/ou étant absorbée par eux, la protéine (i) étant choisie dans le groupe, suivant :
- IgE, Fragment d'IgE, en particulier fragment Fc d'IgE,
- Anticorps contre le récepteur d'IgE des mastocytes et/ou des basophiles, fragment d'anticorps contre le récepteur d'IgE des mastocytes et/ou des basophiles, en particulier fragment Fab,
- Anticorps contre le canal potassique spécifique des mastocytes et le peptide MCD inactif et toutefois liant, et
(ii) une protéase clivant une ou plusieurs protéines de l'appareil de sécrétion des mastocytes et/ou des basophiles

3. Protéine hybride comprenant ou consistant en :
(i) une protéine se liant aux mastocytes et/ou aux basophiles et/ou étant absorbée par eux, et
(ii) une protéase clivant une ou plusieurs protéines de l'appareil de sécrétion des mastocytes et/ou des basophiles, la protéase (ii) étant choisie dans le groupe suivant :
- chaîne Légère d'une toxine botulique de Clostridium, en partie de type A, B, C1, D, E, F ou G ;
- fragment catalytique actif de la chaîne légère d'une toxine botulique de clostridium, en partie de type A, B, C1, D, E, F ou G ;
- chaîne légère de la toxine tétanique (TeNT) ;
- fragment catalytique actif de la chaîne légère de la toxine tétanique (TeNT) ;
- protéase d'IgA de Neisseria gonorrhoeae ; et
- domaine catalytique de la protéase d'IgA de Neisseria gonorrhoeae.

4. Protéine hybride selon la revendication 2 ou 3, **caractérisée en ce que** la protéine (i) est choisie dans le groupe selon la revendication 2 et la protéase (ii) dans le groupe selon la revendication 3.

5. Protéine hybride selon la revendication 4, **caractérisée en ce que** une partie de la protéine hybride est, outre la chaîne légère de la toxine botulique de Clostridium ou de la toxine tétanique, également l'extrémité N-terminale de la chaîne lourde de la toxine respective (fragment H_{N}) ou un fragment de celle-ci.

6. Protéine hybride selon l'une des revendications précédentes pour une utilisation comme médicament en médecine humaine ou vétérinaire.

7. Utilisation de la protéine hybride selon l'une de revendication 1 à 5 pour la fabrication d'un médicament destiné à l'inhibition de la dégranulation des mastocytes.
